# EUROPEAN PATENT SPECIFICATION

(11) **EP 3 530 216 B1**
(45) Date of publication and mention of the grant of the patent: **30.08.2023**
(21) Application number: 19158932.4
(22) Date of filing: 22.02.2019
(51) Int. Cl.: A61B 17/16

(54) **SURGICAL REAMER**
CHIRURGISCHE REIBAHLE
FRAISE CHIRURGICALE

(30) Priority: 23.02.2018 IT 201800003010
(43) Date of publication of application: 28.08.2019
(73) Proprietor: HPF S.R.L., 33034 Fagagna (IT)
(72) Inventor: LUALDI, Tommaso, 33034 Fagagna (IT)
(74) Representative: Petraz, Gilberto Luigi

(56) References cited:
- EP-A1- 3 178 417
- WO-A1-2012/136972
- US-A1- 2016 278 792

## Description

### FIELD OF THE INVENTION

The present invention concerns a milling tool for prosthetic surgery operations, which can be used for example to make a bone seating, for example for the installation of a acetabular prosthesis of the hip, or a shoulder prosthesis or other, associable with a corresponding manipulator device.

### BACKGROUND OF THE INVENTION

In general, milling tools are known, which can be used during prosthetic surgery operations and are conformed to make coordinated and mating bone seatings suitable for the disposition and implantation of corresponding components of surgical prostheses.

In particular, milling tools are known, which can be used to make the hemispherical seatings, or in any case with a spherical cap, suitable for the installation of coordinated acetabular cups of hip prostheses.

Known milling tools generally provide a milling cap, hollow inside and having sizes correlated to the bone seating to be made and on which a plurality of through apertures is made, provided with sharp and protruding edges, to perform a mechanical excavation action on the bone.

In this way, by rotating around an axis of rotation or milling, and by performing a mechanical removal action of the bone material, this type of milling tools produce on the bone an impression of the desired size and conformation, substantially corresponding to the milling cap.

It is known that said milling tools are operatively associated with manipulator devices, which can be either manual or automatic.

It is also known that, after each operation, said milling tools must be subjected to washing and sterilization operations, also using washing and sterilization machines intended for this purpose.

The washing and sterilization operations naturally entail an increase in the overall costs deriving from the use of said known instruments or tools.

Furthermore, it is possible that the washing and sterilization operations are sometimes found to be insufficient, so that contaminants, such as viruses, bacteria or suchlike, can also remain adherent particularly to the milling cap and naturally can cause problems for the patient.

These disadvantages related to the effectiveness of the washing and sterilization operations are also due to a certain structural complexity with which the milling tools are made.

Furthermore, the costs of supplying known milling tools, and also the storage costs of these tools, are usually and undesirably high.

Documents EP-A-2.478.852, US-A-2016/0089158 and EP-A-1.764.046 describe milling instruments for orthopedic surgery, in particular for prosthetic surgery, of a known type.

WO2012136972 discloses a milling tool according to the preamble of claim 1.

Other limitations and disadvantages of conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

There is therefore a need to perfect a milling tool for prosthetic surgery operations that can overcome at least one of the disadvantages of the state of the art.

One purpose of the present invention is therefore to provide a milling tool for prosthetic surgery operations which allows to drastically reduce the risk of contamination by external agents, such as viruses, bacteria or suchlike, thus allowing to reduce the risk of contamination for the patient.

Another purpose of the present invention is to provide a milling tool which allows to limit other washing and sterilization operations, to be carried out only on certain zones or parts of the milling tool, thus optimizing the washing and sterilization operations, and even to eliminate them, at least for the operating cutting parts of the milling tool.

Another purpose of the present invention is to provide a milling tool for prosthetic surgery operations that is simple and compact in shape, thus allowing production and supply at lower costs compared to what happens with known milling tools.

Another purpose of the invention is to provide a milling tool which is simple, effective and which allows to carry out the milling operations for which it is intended in an optimal and precise manner.

The Applicant has devised, tested and embodied the present invention to overcome the shortcomings of the state of the art and to obtain these and other purposes and advantages.

### SUMMARY OF THE INVENTION

The present invention is set forth and characterized in the independent claim, while the dependent claims describe other characteristics of the invention or variants to the main inventive idea.

According to the invention, a milling tool for prosthetic surgery operations, is provided, which comprises:
a support part substantially semi-spherical in shape and made of metal, provided with a cutting part having a plurality of protruding cutting elements,
an attachment part made of metal and permanently connected to the support part by welding,
an attachment head provided with clamping means able to be selectively activated for a stable and releasable connection to the attachment part, said attachment head being able to be connected to a manipulator device.

The attachment part is provided with a central connection seating, having a polygonal shape, in particular quadrangular, in order to allow a selectively releasable connection with the attachment head; the attachment part also comprises spokes between which through apertures 30 are made.

According to possible embodiments, at least the cutting part is made of titanium.

According to possible embodiments, the support part is made of titanium.

According to other possible embodiments, the attachment part is made of titanium.

In accordance with other embodiments, the milling tool is entirely made of titanium.

In accordance with other embodiments, the cutting part and the support part are made in a single body with each other.

In accordance with other embodiments, the support part has a thickness comprised between 0.8 mm and 1.2 mm.

In accordance with other embodiments, the attachment part has a thickness comprised between 0.9 and 1.1 mm.

In accordance with other embodiments, the attachment head has a polygonal section shape, in particular quadrangular, mating with the shape of the connection seating and the clamping means are provided with a retractable elastic element configured to selectively engage the attachment part in correspondence with the connection seating.

In accordance with other embodiments, the attachment part comprises a peripheral band with a continuous annular shape and three central spokes angularly spaced substantially equally.

In accordance with other embodiments, the attachment part is formed by a bar provided with the central connection seating and defining only two diametrically opposite spokes.

In accordance with other embodiments, the attachment head is provided with a protruding annular bead abutting against the attachment part.

In accordance with other embodiments, the attachment part has an edge, or crown, with a perimeter step edge with an annular shape to couple and abut with an annular peripheral edge of the support part, where the attachment part is welded to the support part; the perimeter step edge defines an annular portion to restrict the section of the attachment part embedded inside the support part and an annular abutment portion coupled head-wise with an annular peripheral external edge of the support part.

In accordance with other embodiments, the attachment part has a smooth peripheral edge coupling with the support part welded and completely embedded, flush with an annular peripheral external edge of the support part.

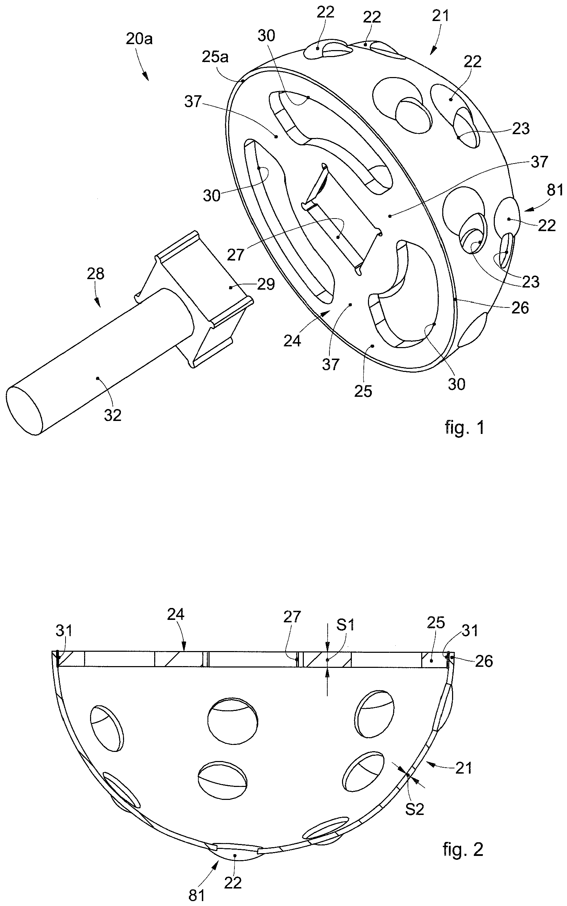

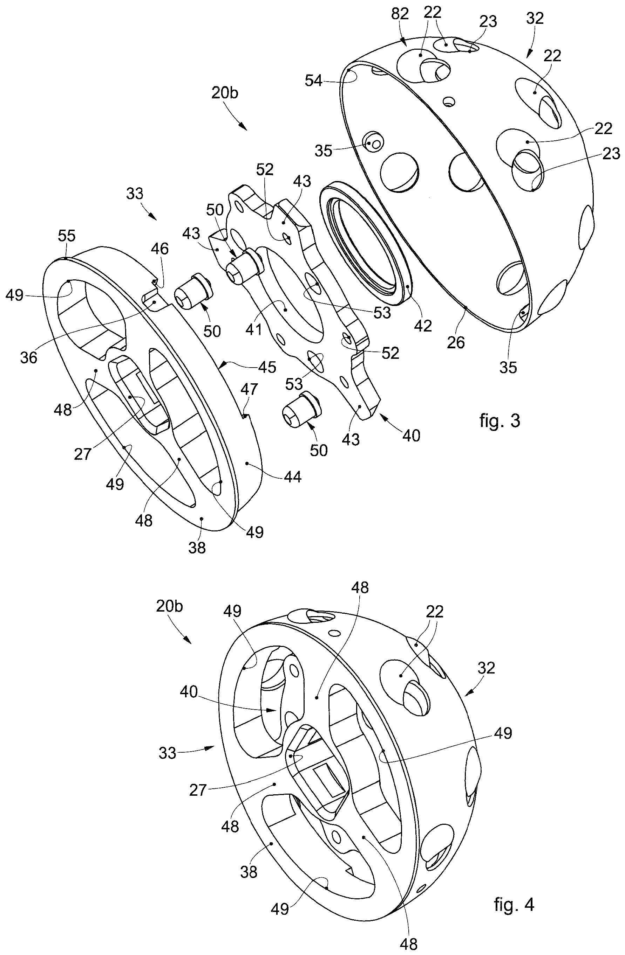

These and other aspects, characteristics and advantages of the present disclosure will be better understood with reference to the following description, drawings and attached claims. The drawings, which are integrated and form part of the present description, show some embodiments of the invention in figures 1, 2 and 11-16, whereas figures 3-10 show exemplary milling tools not forming part of the claimed invention.

The various aspects and characteristics described in the present description can be applied individually where possible.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a three-dimensional view of a first executive embodiment of a milling tool according to the present invention;
- fig. 2 is a section view of a support part of the milling tool of fig. 1;
- fig. 3 is a three-dimensional and exploded view of a second executive embodiment of a milling tool not forming part of the claimed invention;
- fig. 4 is a three-dimensional and assembled view of the second executive embodiment of the milling tool of fig. 3;
- fig. 5 is a front view of the second executive embodiment of the milling tool of fig. 4;
- fig. 6 is a section view of the second executive embodiment of the milling tool considered according to the section line VI-VI of fig. 5;
- fig. 7 is a another three-dimensional view of the second executive embodiment of the milling tool;
- fig. 8 is a three-dimensional and exploded view of a third executive embodiment of a milling tool not forming part of the claimed invention;
- fig. 9 is a longitudinal and exploded section view of the third executive embodiment of the milling tool;
- fig. 10 is a three-dimensional and assembled view of the third executive embodiment of the milling tool;
- fig. 11 is a lateral view of a milling tool in accordance with some embodiments described herein;
- fig. 12 is a section along line XII-XII of fig. 11;
- fig. 13 is a lateral view with separate parts of a milling tool in accordance with some embodiments described here;
- fig. 14 is a section along the line XIII-XIII of fig. 13;
- fig. 15 is a perspective view with separate parts of a milling tool in accordance with embodiments described here;
- fig. 16 is a perspective view with separate parts of a milling tool in accordance with other embodiments described here.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We will now refer in detail to the various embodiments of the present invention shown in figures 1, 2 and 11-16; and to the exemplary milling tools not forming part of the claimed invention shown in figures 3-10.

Each example is supplied by way of illustration of the invention and shall not be understood as a limitation thereof. The scope of the invention is defined in the appended claims.

Embodiments described with reference to the attached drawings concern a milling tool 20a, 20b, 20c, 20d, 20e for prosthetic surgery, in particular for making concave seatings for the implantation of prostheses, for example of the shoulder or of the hip. The milling tool comprises:
- a support part 21, 32 having a substantially semispherical shape, that is, shaped as a hemispherical cap, and made of metal, provided, in a single body, with a cutting part 81, 82 having a plurality of protruding cutting elements 22, or blades;
- an attachment part 24 made of metal and permanently connected to the support part 21, 32 by welding 31,
- an attachment head 29 provided with clamping means 84 that can be selectively activated to obtain a stable and releasable connection to the attachment part 24, the attachment head 29 being able to be connected to a manipulator device 28.

The attachment part 24 is provided with a central connection seating 27, having a polygonal shape, in particular quadrangular, to allow a selectively releasable connection with the attachment head 29.

The polygonal shape, in particular quadrangular shape, of the central connection seating 27 is advantageous to obtain the effective transmission of the twisting torque needed for the rotation of the milling tool in question.

Advantageously, at least the cutting part is made of titanium.

In possible implementations, the support part 21, 32 is made of titanium.

In other possible implementations, the attachment part 24 is made of titanium.

Advantageously, the milling tool 20a, 20b, 20c, 20d, 20e can be entirely made of titanium.

Titanium is particularly advantageous as it is hypoallergenic and biocompatible.

Alternatively, one, more or all of the above components can be made of steel.

In the embodiments described using figs. 1, 2, 3, 4, 11, 12, 13, 14, 15 and 16, the cutting part 81, 82 and the support part 21, 32 are made in a single body.

In possible implementations, the support part 21 has a thickness S2 comprised between 0.8 mm and 1.2 mm.

In other possible implementations, the attachment part 24 has a thickness S1 comprised between 0.9 mm and 1.1 mm.

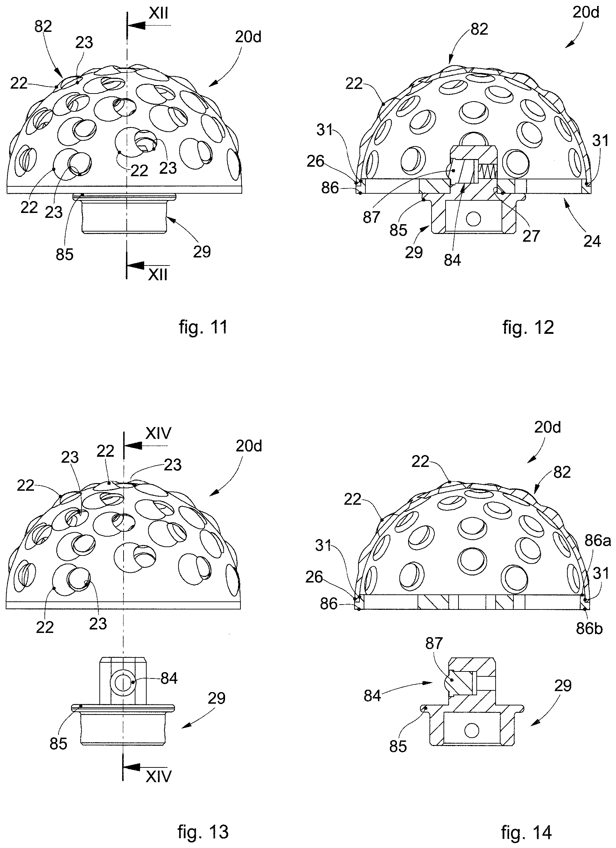

In possible embodiments described using figs. 1, 2, 11, 12, 13, 14, 15 and 16, the attachment head 29 has a polygonal section shape, in particular quadrangular, mating with the shape of the connection seating 27, and the clamping means 84 are provided with an elastic retractable element 87 configured to selectively engage the attachment part 24 in correspondence with the connection seating 27 (see fig. 12 for example).

The attachment head 29 can be provided for connection to a manipulator device 28.

The elastic retractable element 87 therefore acts as an advantageous selective clamping element with the attachment part 24. This is advantageously useful, for example, to prevent unwanted detachment or uncoupling of the attachment part 24 and the attachment head 29 associated with the manipulator device 28.

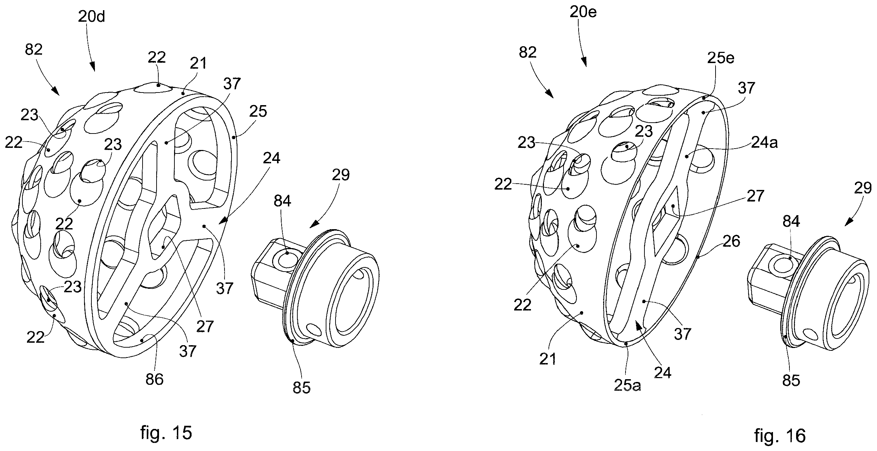

In possible embodiments, the attachment part 24 comprises a peripheral band 25 with a continuous annular shape and three central spokes or arms 37, angularly spaced substantially equally (see figs. 1 and 15 for example).

In other possible embodiments, the attachment part 24 is formed by a bar or crosspiece 24a, provided with the central connection seating 27 and defining only two diametrically opposite spokes or arms 37 (see fig. 16 for example).

In possible embodiments described using figs. from 11 to 16, the attachment head 29 is provided with a protruding annular bead 85 abutting against the attachment part 24 (see fig. 12 for example). The provision of the protruding annular bead 85 is advantageous in that it defines an end-of-travel or safety abutment for the connection between the attachment head 29 and the attachment part 24, also preventing situations in which, by exerting thrust and pressure on the manipulator device 28, the attachment head 29 is thrust excessively and undesirably beyond the attachment part 24, toward the inside of the support part 21, with the evident risks of such a case.

In possible embodiments described using figs. 11 to 15, the attachment portion 24 has a perimeter step edge or crown 86, with an annular shape to couple and abut with an annular peripheral edge 26 of the support part 21, where the attachment part 24 is welded to the support part 21 by means of the welding 31; the perimeter step edge 86 defines an annular portion 86a to restrict the section of the attachment part 24 embedded inside the support part 21, and an annular abutment portion 86b coupled head-wise with an annular peripheral external edge 26 of the support part 21.

In other possible embodiments described using fig. 16, the attachment part 24 has a smooth peripheral edge 25a to couple with the support part 21, welded and completely embedded, flush with an annular peripheral external edge 26 of the support part 21.

Other embodiments described using figs. 1 to 16 concern milling tools 20a, 20b, 20c, 20d, 20e for prosthetic surgery operations.

The milling tools 20a, 20b, 20c, 20d, 20e comprise:
- a support part 21, 32, 57 with a substantially hemispherical shape,
- an attachment part 24, 33, 68 associable with the support part 21, 32, 57 to allow the selectively releasable connection of a manipulator device 28; and
- a cutting part 81, 82, 83 associated at least with the support part 21, 32, 57 and provided with one or more cutting elements 22.

According to possible embodiments, at least the cutting part 81, 82, 83 can be single-use, and therefore of a type that can be replaced after each use of the milling tool 20a, 20b and 20c. According to a possible solution, at least the cutting part 81, 82, 83 is made of titanium.

The use of this material, also given its relatively low cost, allows to make at least the cutting part 81, 82, 83 single-use. Furthermore, the use of titanium ensures the high biocompatibility of this material with the human body, preventing problems of postoperative rejection.

In other embodiments, on the contrary, at least the cutting part 81, 82, 83 is reusable several times, and therefore is not single-use.

The cutting part 81, 82 and 83 can comprise a plurality of cutting elements 22, or blades, provided to perform the action of removing material.

In accordance with another embodiment of the present invention, the support part 21, 32, 57 can have a semispherical cap shape on which the cutting part 81, 82, 83 is associated.

The attachment part 24 comprises at least one central connection seating 27 to allow the selectively releasable connection of the manipulator device 28.

The manipulator device 28 can be provided with an attachment head 29 with a shape mating with the central connection seating 27, in particular to engage the attachment head 29, and a handle 34.

In accordance with variant embodiments, the manipulator device 28 can be provided with hooking portions to engage in corresponding hooking elements provided in the attachment part 24.

With reference to the attached drawings, possible embodiments of milling tools are shown, indicated respectively by the reference numbers 20a, 20b, 20c, 20d, 20e.

In particular, in one embodiment of the present invention (figs. 1, 2 and 11-16) the milling tool 20a, 20d, 20e for prosthetic surgery operations, according to the invention, provides that the support part 21, the cutting part 81, and the support part 32 are irremovably connected to each other so as to define overall a completely single-use single body.

According to one embodiment (figs. 1, 2 and 11-16), the cutting part 81 and the support part 21 can be made in a single body.

According to another solution (figs. 1, 2, 11-16), the attachment part 24 and the support part 21 with the corresponding cutting part 81 are made in one piece, that is, in a single body, or can be joined together permanently, by welding, for example, so as to constitute a single single-use assembly.

In accordance with possible solutions of the present invention (figs. 1, 2, 11-16), the attachment part 24, the support part 21 and the cutting part 81 can all be made of titanium making the whole milling tool 20a, 20d, 20e completely single-use and biocompatible with the interventions of the operator. In possible solutions (figs. 1, 2 and 11-16), the support part 21 has a substantially semispherical shape and is hollow inside. The cutting elements 22, preferably, can be uniformly distributed on the surface of the support part 21 and through apertures 23 are made in correspondence therewith.

The cutting elements 22 can have various shapes and have the function of cutting or milling the bone component, while the through apertures 23 allow the removal of the bone residues from the cutting or milling zone.

According to a possible solution, the cutting elements 22 defined above are made in the thickness of the support part 21 and each is provided with at least one cutting edge to allow to remove the material.

The support part 21 is provided with an end annular edge 26 having a circular shape in which the attachment part 24 is irremovably connected.

According to one solution (figs. 1, 2 and 11-16), the attachment part 24 has a discoidal shape and is provided with an annular or peripheral band 25 with a shape and size mating with the peripheral annular edge 26 of the support part 21. In particular, it can be provided that the annular band 25 of the attachment part 24 is in contact and fixedly attached to the peripheral annular edge 26 of the support part 21.

In particular, the annular band 25 of the attachment part 24 can be positioned in part of the semi-spherical cavity defined by the support part 21, that is, it can be at least partly embedded therein. This positioning of the attachment part 24 allows to obtain an increase in the containing rigidity of the support part 21, preventing a collapse thereof in correspondence with the annular edge 26.

According to the invention, the attachment part 24 comprises spokes or arms 37 (see figs. 1, 2 and 11-16) between which through apertures 30 are made, which have a lightening function and allow to remove the bone residues from the cutting or milling zone.

In accordance with some solutions (figs. 1, 2 and 11-16), the attachment part 24 has a thickness S1 comprised between 0.9 mm and 1.1 mm, and preferably about 1 mm, see fig. 2. These thicknesses are sufficient to allow to transmit the twisting torque exerted by the manipulator device 28, preventing damage to the attachment part 24 and also making it single-use, thus reducing the waste of material.

The support part 21, according to possible embodiments with reference, for example, to figs. 1 and 2, which as we said can have the shape of a hemispherical cap, can have a thickness S2 comprised between 0.3 mm and 0.8 mm and preferably about 0.5 mm.

It has been found that these values of thickness S2 of the support part 21 guarantee a perfect circularity of the annular edge 26 of the support part 21, also during use of the milling tool 20a.

In other embodiments, as we said, the thickness S2 of the support part 21 can be comprised between 0.8 mm and 1.2 mm and preferably about 1 mm, particularly when the support part 21 and the corresponding cutting part 81, 82 are reusable several times as described above, that is, they are not single-use.

The annular band 25 of the attachment part 24 and the annular edge 26 of the support part 21 are joined by welding according to the invention.

The milling tool 20a, 20d, 20e therefore has a support part 21 with a cutting part 81 and an attachment part 24 which can be reused several times, that is, they are not single-use, or they can be completely single-use, that is, they are replaced after each use, as required.

In another embodiment not forming part of the claimed invention (figs. 3-7) the milling tool 20b comprises the support part 32 provided with the cutting part 82 and the attachment part 33.

In this case too, by way of example, the cutting part 82 and the support part 32 can be made in a single body.

In particular, it can be provided that the cutting part 82 is formed by the cutting elements 22, variously positioned and made on the support part 32.

In this case the support part 32 and the cutting part 82 are single-use, while the attachment part 33 can be reusable and therefore can be selectively connected removably to the support part 32.

The support part 32 is shaped like a hemispherical cap and therefore comprises the annular edge 26 which defines a circular aperture 54 in which to insert the attachment part 33.

The support part 32 and the attachment part 33 are provided with selectively releasable connection means, to allow the selective connection and subsequent separation, for example after use, of the support part 32 with respect to the attachment part 33.

The support part 32 comprises internally, that is, in the concavity defined by the hemispherical cap, a plurality of radially protruding teeth 35 configured to engage with corresponding seatings 36 made in a disc 38 of the attachment part 33.

The disc 38 comprises a protruding annular edge 55 and on which the annular edge 26 of the support part 32 abuts.

The teeth 35 are preferably made in proximity to the annular edge 26 of the support part 32.

The teeth 35 can be formed by plastic deformation of the support part 32 to generate a protrusion of the hemispherical cap toward the inside.

For example, it is possible to provide three teeth 35, disposed equally distanced from each other in proximity to the annular edge 26 of the support part 32.

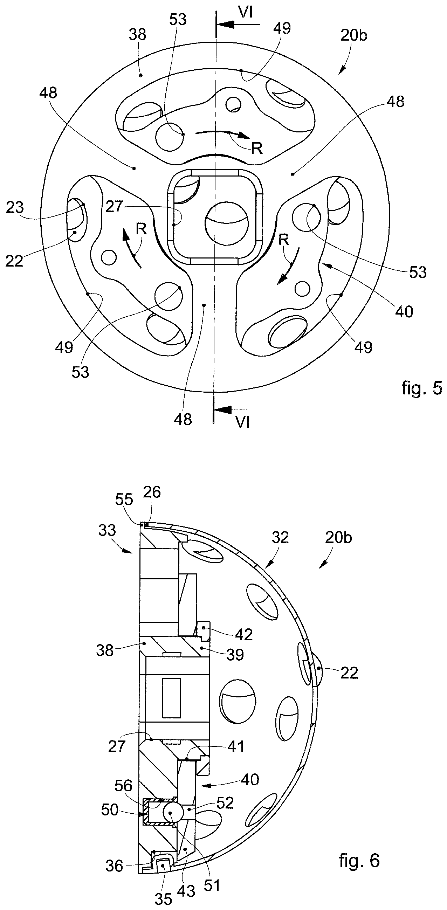

The disc 38, see fig. 6 in particular, comprises a cylindrical support 39 located, during use, in the cavity of the spherical cap of the support part 32.

A rotatable clamping element 40 is positioned on the cylindrical support 39, and is provided for this purpose with a through hole 41 in which the cylindrical support 39 is inserted.

The cylindrical support 39 can be threaded to the protruding end, so as to engage with an internally threaded closing ring 42.

Once the clamping element 40 has been inserted on the cylindrical support 39, the ring 42 is screwed to the cylindrical support 39, so as to keep the clamping element 40 in the correct position and allow it to rotate with respect to the cylindrical support 39 and thus to the disc 38.

The clamping element 40 comprises a plurality of radially protruding spokes 43, and having an extension such as to reach, in a clamping position, in correspondence with the seatings 36 made on the disc 38.

Each of the spokes 43 has the function of preventing the separation of the attachment part 33 from the support part 32, once the seatings 36 of the disc 38 have been correctly positioned on the respective teeth 35 as shown in fig. 6.

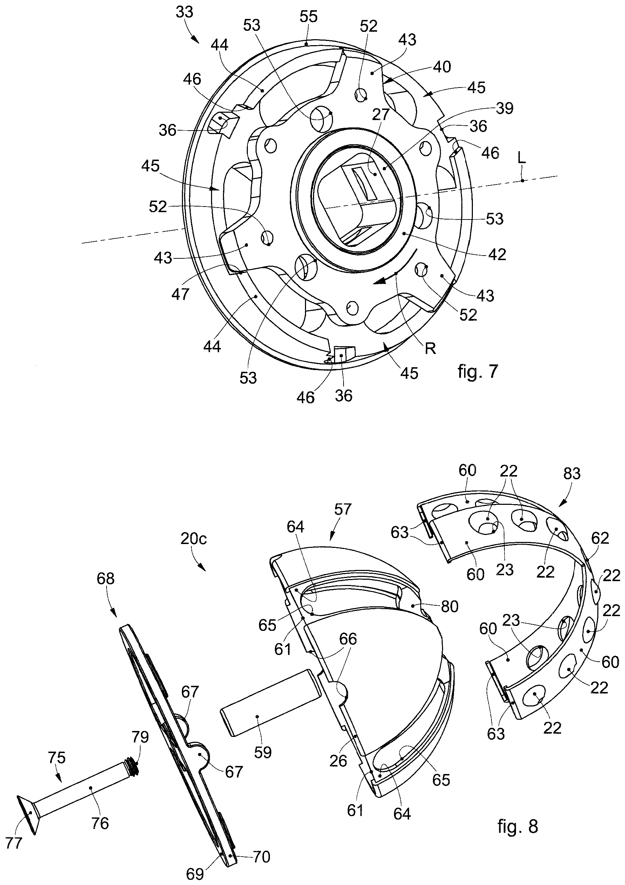

With particular reference to fig. 7, it can be seen that on the lateral wall 44 of the disc 38 arched grooves 45 are made, along which the spokes 43 can be rotated, integral with the clamping element 40.

The clamping element 40 is therefore substantially rotatable with respect to an axis L to effect a rotation in the direction R, in one sense or the other, by an amplitude defined by the extension of the arched grooves 45.

Each of the arched grooves 45 comprises two end-of-travel ends 46 and 47 which define two limit positions of the spokes 43 of the clamping element 40: an active position in which each spoke 43 is in correspondence with the seating 36 of the disc 38, and an inactive position in which the clamping element 40 is inoperative and allows the reciprocal separation of the attachment part 33 with respect to the support part 32.

The disc 38 also comprises spokes 48 between which through apertures 49 are made.

The through apertures 49 allow to interact from the outside with the clamping element 40 so as to be able to rotate it at least into the active position and at least into the inactive position.

Seatings 56 are provided in the spokes 48, see the section in fig. 6, to house a series of pins 50 to position the clamping element 40.

The pins 50 are provided with a spherical or semi-spherical head 51 suitable to engage, for example in snap-in mode, in corresponding holes 52 made on the spokes 43 of the clamping element 40, in particular in the position in which the spokes 43 are located in correspondence with the seatings 36 of the disc 38, thus in an active clamping position.

The pins 50 allow to keep the clamping element 40 securely in place at least when it is in the active clamping position.

Other holes 53 could be made on the clamping element 40, suitable to allow engagement with a rotating tool or device, or suchlike.

If we observe fig. 5 for example, it can be assumed that, from the outside of the milling tool 20b, a suitable tool can be inserted through the through apertures 49 and engage in the holes 53 to allow the rotation R of the clamping element 40.

Substantially therefore, the disc 38 of the attachment part 33 can already be supplied with the clamping element 40 mounted and rotatable in one direction or the other with respect to the disc 38, as a function of the extension of the arched grooves 45. The disc 38 and the clamping element 40 are kept adjacent and in position thanks to the ring 42.

The attachment part 33 with the disc 38, the clamping element 40 and the ring 42 appears, for example, as in fig. 7, so that the seatings 36 are not covered by the spokes 43 of the clamping element 40.

The attachment part 33 is then inserted into the support part 32 through the aperture 54 and the seatings 36 of the disc 38 are positioned on the teeth 35.

The insertion of the attachment part 33 is completed when the annular edge 55 abuts on the annular edge 26 of the support part 32 and the seatings 36 are completely positioned on the teeth 35.

When positioning has been completed, as for example in fig. 4, the clamping element 40 is rotated in direction R, fig. 7, so that the spokes 43 move into correspondence with the seatings 36, so that the teeth 35 will be positioned between the seatings 36 and the spokes 43, see the cross-section in fig. 6 for example: by means of the spokes 43, the clamping element 40 abuts with the teeth 35, preventing the extraction of the attachment part 33.

In this active or clamping position, the clamping element 40 therefore interferes with the teeth 35, in particular by means of the spokes 43.

The attachment part 33, in this position, is firmly connected to the support part 32 and therefore cannot be disengaged from it. The correct positioning of the attachment part 33 on the support part 32 is also guaranteed by the engagement of the holes 52 of the spokes 43 on the pins 50.

The disc 38 provided with a rotatable clamping element 40 therefore represents a non-restrictive example of possible releasable connection means of the attachment part 33 to the support part 32.

In order to separate the attachment part 33 from the support part 32, the clamping element 40 will be rotated in the opposite direction to the clamping rotation R, so that the spokes 43 no longer abut on the tooth 35 of the support part 32.

In this embodiment of the milling tool 20b, therefore, the support part 32 and the cutting part 82 are single-use, while the attachment part 33, comprising the disc 38 and the clamping element 40, is reusable.

Fig. 8, fig. 9 and fig. 10 show another embodiment of the milling tool 20c.

The milling tool 20c comprises the support part 57 in the form of a hemispherical cap configured to house the cutting part 83.

Cutting elements, such as for example the cutting elements 22 of fig. 1 or fig. 3, can be positioned or made on the cutting part 83, in proximity to the through apertures 23.

In this embodiment of the milling tool 20c, only the cutting part 83 is single-use, while the other parts of the milling tool 20c are reusable.

The cutting part 83 is formed by a plurality of arcuate-shaped branches 60, configured to position themselves in corresponding arched seatings 64 made on the hemispherical surface of the support part 57.

The branches 60 are joined to a common end 62 and are provided, at the free ends, with an edge 63, bent toward the inside of the milling tool 20c, in particular in a radial direction.

It is possible to provide that the cutting part 83 is formed, for example, by four reciprocally equidistant branches 60.

The seatings 64, configured to house the branches 60 to size, can preferably be provided with through apertures 65, located during use in correspondence with the internal side of the cutting elements 22. The through apertures 65 allow to discharge the material which is removed by the cutting elements 22 in the concavity of the spherical cap.

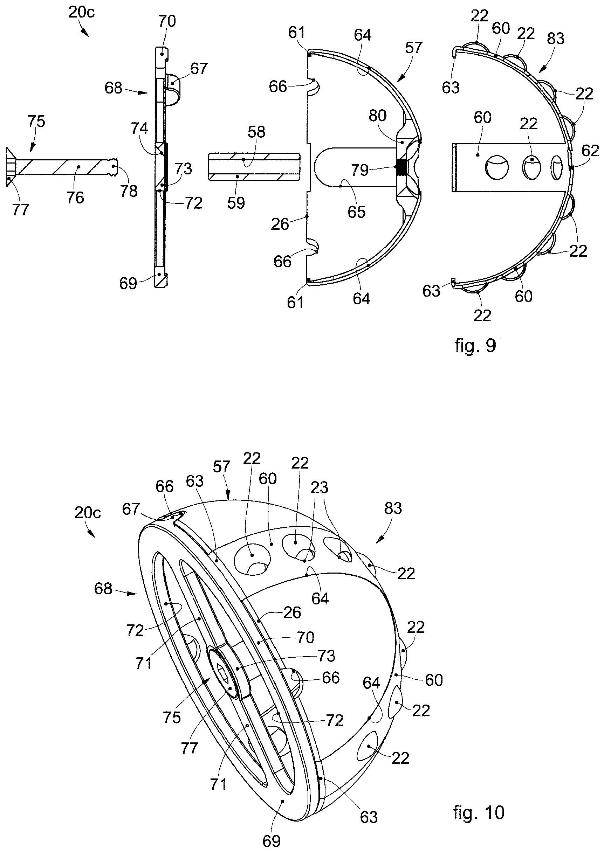

Each of the seatings 64 comprises an edge 61, in which the edge 63 of the corresponding branch 60 is positioned, so that the cutting part 83 is correctly positioned on the support part 57, as for example in fig. 10.

To this purpose, in the positioning of the cutting part 83 on the support part 57, the branches 60 widen slightly and elastically, until the edges 63 are correctly positioned under the edges 61 of the seatings 64.

The support part 57 is also provided, on the annular edge 26, with one or more notches 66, for example of an arcuate shape, configured to engage with elements 67 protruding from an attachment part 68.

The attachment part 68 comprises a disc 69 on whose annular edge 70 the protruding elements 67 are made.

The protruding elements 67 are inserted into the notches 66 of the support part 57, so as to guarantee the correct positioning of the attachment part 68 in the support part 57 and in such a way as to prevent any reciprocal rotation thereof.

The disc 69 of the attachment part 68 comprises spokes 71 between which through apertures 72 are made and joined in a central part 73.

The central part 73 is provided with a through hole 74, suitable to allow the passage of the shaft 76 of a pin 75 and to house the head 77 to size.

The head 77 can have a truncated cone shape and the through hole 74 can consequently be provided with a corresponding truncated cone shape.

The shaft 76 of the pin 75 comprises, at the opposite end of that of the head 77, a threaded part 78, configured to screw with a corresponding threaded part 79 made inside the support part 57, in particular in a top portion 80 of the support part 57.

The attachment part 68 also comprises a spacer element 59 provided with a through hole 58 and suitable to rest on one side on the central part 73 of the disc 69 and, on the other side, on the top portion 80 of the support part 57.

When assembling the milling tool 20c, first of all, preferably, the cutting part 83 is positioned on the support part 57, therefore with the branches 60 correctly positioned in the seatings 64.

Then, the attachment part 68 is assembled and connected to the support part 57.

The shaft 76 of the pin 75 is inserted into the through hole 74 of the disc 69 and then into the through hole 58 of the spacer element 59, so that the threaded part 78 protrudes from the spacer element 59 and can engage with the threaded portion 79 of the top portion 80 of the support part 57.

The protruding elements 67 of the disc 69 are naturally inserted in the corresponding notches 66.

The annular edge 70 of the disc 69 also abuts against at least the annular edge 26 of the support part 57.

In its assembled and operative configuration, the milling tool 20c will therefore appear as in fig. 10.

The disc 69 associable with the pin 75 which can be screwed into the support part 57 therefore represents a non-restrictive example of possible releasable connection means of the attachment part 68 to the support part 57.

A corresponding manipulator device can be connected to the disc 69 of the attachment part 68, and is provided with suitable releasable connection elements, for example, to the spokes 71 of the disc 69.

The milling tools 20a, 20b, 20c, 20d, 20e can therefore have at least the cutting parts 81, 82, 83 of a single-use type, or which can be reused, and therefore not single-use.

In the case of the milling tool 20a, 20d, 20e, the support part 21 and the attachment part 24 can also be single-use, or reusable, and therefore not single-use. In the case of the milling tool 20b, in addition to the cutting part 82, the support part 32 can also be single-use. In the case of the milling tool 20c, only the cutting part 83 can be single-use.

The present milling tool 20a, 20b, 20c, 20d, 20e is preferably made of titanium, or at least the support parts 21, 32, 57 and the cutting parts 81, 82, 83, so as to be biocompatible with the organism and avoid problems of rejection for the patient. In particular, the titanium used can be grade 0 or 1.

Furthermore, titanium guarantees high mechanical resistance in ratio to weight, which can be advantageously contained.

It is clear that modifications and/or additions of parts can be made to the milling tool 20a, 20d, 20e for prosthetic surgery operations as described heretofore, without departing from the scope of the present invention, which is defined by the appended claims.

It is also clear that, although the present invention has been described with reference to some specific examples, a person of skill in the art shall certainly be able to achieve many other equivalent forms of milling tool 20a, 20d, 20e for prosthetic surgery operations, having the characteristics as set forth in the claims and hence all coming within the field of protection defined thereby.

Although the above refers to embodiments of the invention, other embodiments can be provided without departing from the scope of protection, which is defined by the following claims.

## Claims

1. Milling tool for prosthetic surgery operations, comprising:
a support part (21, 32) substantially semi-spherical in shape and made of metal, provided with a cutting part (81, 82) having a plurality of protruding cutting elements (22),
an attachment part (24) made of metal and permanently connected to said support part (21, 32) by welding (31),
an attachment head (29) provided with clamping means (84) configured to be selectively activated for a stable and releasable connection to said attachment part (24), said attachment head (29) being configured to be connected to a manipulator device (28),
said attachment part (24) being provided with a central connection seating (27), having a polygonal shape, configured to allow a selectively releasable connection with said attachment head (29), **characterized in that** said attachment part (24) further comprises spokes (37) between which through apertures (30) are made.

2. Milling tool as in claim 1, wherein at least the cutting part (81, 82) is made of titanium.

3. Milling tool as in claim 1 or 2, wherein the support part (21, 32) is made of titanium.

4. Milling tool as in claim 1, 2 or 3, wherein the attachment part (24) is made of titanium.

5. Milling tool as in any claim hereinbefore, wherein it is entirely made of titanium.

6. Milling tool as in any claim hereinbefore, wherein said cutting part (81, 82) and said support part (21, 32) are made in a single body with each other.

7. Milling tool as in any claim hereinbefore, wherein said support part (21) has a thickness (S2) comprised between 0.8 mm and 1.2 mm.

8. Milling tool as in any claim hereinbefore, wherein said attachment part (24) has a thickness (S1) comprised between 0.9 and 1.1 mm.

9. Milling tool as in any claim hereinbefore, wherein said attachment head (29) has a polygonal section shape, configured to mate with the shape of said connection seating (27) and said clamping means (84) comprise a retractable elastic element (84) configured to selectively engage the attachment part (24) in correspondence with the connection seating (27).

10. Milling tool as in any claim hereinbefore, wherein said attachment part (24) comprises a peripheral band (25) with a continuous annular shape and three central spokes (37) angularly spaced substantially equally.

11. Milling tool as in any claim from 1 to 9, wherein said attachment part (24) is formed by a bar provided with said central connection seating (27) and defining only two diametrically opposite spokes (37).

12. Milling tool as in any claim hereinbefore, wherein said attachment head (29) is provided with a protruding annular bead (85) abutting against said attachment part (24).

13. Milling tool as in any claim hereinbefore, wherein said attachment part (24) has an edge with perimeter step edge (86) with an annular shape to couple and abut with an annular peripheral edge (26) of said support part (21), where said attachment part (24) is welded to said support part (21), the perimeter step edge (86) defines an annular portion (86a) to restrict the section of said attachment part (24) embedded inside said support part (21) and an annular abutment portion (86b) coupled head-wise with an annular peripheral external edge (26) of said support part (21).

14. Milling tool as in any of the claims from 1 to 12, wherein said attachment part (24) has a smooth peripheral edge (25a) coupling with said support part (21) welded and completely encased, flush with an external annular peripheral edge (26) of said support part (21).

## Patentansprüche

1. Fräswerkzeug für Prothesenchirurgie-Operationen, das Folgendes umfasst:
einen Stützteil (21, 32), der im Wesentlichen von halbkugeliger Form und aus Metall hergestellt ist, wobei er mit einem Schneidteil (81, 82) versehen ist, der eine Vielzahl von vorspringenden Schneidelementen (22) aufweist,
einen Befestigungsteil (24), der aus Metall hergestellt und durch Schweißen (31) dauerhaft mit dem Stützteil (21, 32) verbunden ist,
einen Befestigungskopf (29), der mit Klemmmitteln (84) versehen ist, die dafür konfiguriert sind, selektiv für eine stabile und lösbare Verbindung mit dem Befestigungsteil (24) aktiviert zu werden, wobei der Befestigungskopf (29) dafür konfiguriert ist, mit einer Manipulationsvorrichtung (28) verbunden zu werden,
wobei der Befestigungsteil (24) mit einem zentralen Verbindungssitz (27) versehen ist, der eine vieleckige Form aufweist, die dafür konfiguriert ist, eine selektiv lösbare Verbindung mit dem Befestigungskopf (29) zu ermöglichen, **dadurch gekennzeichnet, dass**
der Befestigungsteil (24) ferner Speichen (37) umfasst, zwischen denen Durchgangsöffnungen (30) hergestellt sind.

2. Fräswerkzeug nach Anspruch 1, wobei zumindest der Schneidteil (81, 82) aus Titan hergestellt ist.

3. Fräswerkzeug nach Anspruch 1 oder 2, wobei der Stützteil (21, 32) aus Titan hergestellt ist.

4. Fräswerkzeug nach Anspruch 1, 2 oder 3, wobei der Befestigungsteil (24) aus Titan hergestellt ist.

5. Fräswerkzeug nach einem der vorhergehenden Ansprüche, wobei es vollständig aus Titan hergestellt ist.

6. Fräswerkzeug nach einem der vorhergehenden Ansprüche, wobei der Schneidteil (81, 82) und der Stützteil (21, 32) in einem einzigen Körper miteinander hergestellt sind.

7. Fräswerkzeug nach einem der vorhergehenden Ansprüche, wobei der Stützteil (21) eine Dicke (S2) aufweist, die zwischen 0,8 mm und 1,2 mm umfasst.

8. Fräswerkzeug nach einem der vorhergehenden Ansprüche, wobei der Befestigungsteil (24) eine Dicke (S1) aufweist, die zwischen 0,9 mm und 1,1 mm umfasst.

9. Fräswerkzeug nach einem der vorhergehenden Ansprüche, wobei der Befestigungskopf (29) eine vieleckige Schnittform aufweist, die dafür konfiguriert ist, mit der Form des Verbindungssitzes (27) zusammenzupassen, und die Klemmmittel (84) ein zurückziehbares elastisches Element (84) umfassen, das dafür konfiguriert ist, den Befestigungsteil (24) selektiv in Übereinstimmung mit dem Verbindungssitz (27) in Eingriff zu nehmen.

10. Fräswerkzeug nach einem der vorhergehenden Ansprüche, wobei der Befestigungsteil (24) ein an der Außenseite befindliches Band (25) mit einer durchgehenden ringförmigen Gestalt und drei zentrale Speichen (37), die im Wesentlichen gleichmäßig winklig beabstandet sind, umfasst.

11. Fräswerkzeug nach einem der Ansprüche 1 bis 9, wobei der Befestigungsteil (24) durch einen Riegel ausgebildet ist, der mit dem zentralen Verbindungssitz (27) versehen ist und nur zwei diametral entgegengesetzte Speichen (37) definiert.

12. Fräswerkzeug nach einem der vorhergehenden Ansprüche, wobei der Befestigungskopf (29) mit einer vorspringenden ringförmigen Wulst (85) versehen ist, die an dem Befestigungsteil (24) anliegt.

13. Fräswerkzeug nach einem der vorhergehenden Ansprüche, wobei der Befestigungsteil (24) eine Kante mit einer Umfangsstufenkante (86) mit einer ringförmigen Gestalt aufweist, um mit einer ringförmigen an der Außenseite befindlichen Kante (26) des Stützteils (21) zu koppeln und anzuliegen, wo der Befestigungsteil (24) an den Stützteil (21) geschweißt ist, wobei die Umfangsstufenkante (86) einen ringförmigen Abschnitt (86a), um die Sektion des Befestigungsteils (24), die innerhalb des Stützteils (21) eingebettet ist, zu beschränken, und einen ringförmigen Widerlagerabschnitt (86b), der Kopf an Kopf mit einer ringförmigen an der Außenseite befindlichen Außenkante (26) des Stützteils (21) gekoppelt ist, definiert.

14. Fräswerkzeug nach einem der Ansprüche 1 bis 12, wobei der Befestigungsteil (24) eine glatte an der Außenseite befindliche Kante (25a) aufweist, die mit dem geschweißten und vollständig umschlossenen Stützteil (21) koppelt, bündig mit einer ringförmigen an der Außenseite befindlichen Außenkante (26) des Stützteils (21) .

## Revendications

1. Outil de fraisage pour des opérations de chirurgie prothétique, comprenant :
une pièce de support (21, 32) de forme sensiblement semi-sphérique et réalisée en métal, pourvue d'une partie coupante (81, 82) présentant une pluralité d'éléments coupants saillants (22),
une pièce de fixation (24) faite en métal et fixée de façon permanente à ladite pièce de support (21, 32), par soudure (31),
une tête de fixation (29) munie de moyens de serrage (84) configurés pour être activés de manière sélective afin de permettre une connexion stable et amovible à ladite pièce de fixation (24), ladite tête de fixation (29) étant configurée pour être reliée à un dispositif de manipulation (28),
ladite pièce de fixation (24) étant pourvue d'un siège de raccordement central (27), ayant une forme polygonale, configuré pour permettre une connexion apte à être libérée de manière sélective à ladite tête de fixation (29), **caractérisé en ce que** ladite pièce de fixation (24) comprend en outre des rayons (37) entre lesquels des ouvertures traversantes (30) sont aménagées.

2. Outil de fraisage selon la revendication 1, dans lequel au moins la partie coupante (81,82) est faite de titane.

3. Outil de fraisage selon la revendication 1 ou la revendication 2, dans lequel la pièce de support (21, 32) est faite de titane.

4. Outil de fraisage selon la revendication 1, 2 ou 3, dans lequel la pièce de fixation (24) est faite de titane.

5. Outil de fraisage selon l'une quelconque des revendications précédentes, l'outil étant entièrement fait de titane.

6. Outil de fraisage selon l'une quelconque des revendications précédentes, dans lequel ladite partie coupante (81, 82) et ladite pièce de support (21, 32) sont réalisées en un seul corps l'une avec l'autre.

7. Outil de fraisage selon l'une quelconque des revendications précédentes, dans lequel ladite pièce de support (21) a une épaisseur (S2) comprise entre 0,8 mm et 1,2 mm.

8. Outil de fraisage selon l'une quelconque des revendications précédentes, dans lequel ladite pièce de fixation (24) a une épaisseur (S1) comprise entre 0,9 et 1,1 mm.

9. Outil de fraisage selon l'une quelconque des revendications précédentes, dans lequel ladite tête de fixation (29) a une forme de section polygonale, configurée pour coopérer avec la forme dudit siège de connexion (27), et lesdits moyens de serrage (84) comprennent un élément élastique rétractable (84) configuré pour venir en engagement de manière sélective avec la pièce de fixation (24) en correspondance avec le siège de connexion (27).

10. Outil de fraisage selon l'une quelconque des revendications précédentes, dans lequel ladite pièce de fixation (24) comprend une bande périphérique (25) de forme annulaire continue et trois rayons centraux (37) espacés angulairement de façon sensiblement égale.

11. Outil de fraisage selon l'une quelconque des revendications 1 à 9, dans lequel ladite pièce de fixation (24) est formée par une barre munie dudit logement central de raccordement (27) et définissant seulement deux rayons (37) diamétralement opposés.

12. Outil de fraisage selon l'une quelconque des revendications précédentes, dans lequel ladite tête de fixation (29) est pourvue d'une pièce annulaire saillante (85) venant en butée contre ladite pièce de fixation (24).

13. Outil de fraisage selon l'une quelconque des revendications précédentes, dans lequel ladite pièce de fixation (24) a un bord avec un bord périmétrique à crans (86) ayant une forme annulaire afin de venir en accouplement avec, et de buter contre, un bord périphérique annulaire (26) de ladite pièce de support (21), ladite pièce de fixation (24) étant soudée à ladite pièce de support (21), le bord périmétrique à crans (86) définit une partie annulaire (86a) pour restreindre la section de ladite pièce de fixation (24) encastrée à l'intérieur de ladite pièce de support (21) et une partie de butée annulaire (86b) reliée par la tête à un bord externe périphérique annulaire (26) de ladite pièce de support (21).

14. Outil de fraisage selon l'une quelconque des revendications 1 à 12, dans lequel ladite pièce de fixation (24) a un bord périphérique lisse (25a) relié à ladite pièce de support (21) soudé et complètement emboîté, affleurant avec un bord périphérique annulaire externe (26) de ladite pièce de support (21).
